# EUROPEAN PATENT SPECIFICATION

(11) **EP 1 813 217 B1**
(45) Date of publication and mention of the grant of the patent: **13.10.2010**
(21) Application number: 06001882.7
(22) Date of filing: 30.01.2006
(51) Int. Cl.: A61B 17/00, A61F 2/46

(54) **Device for injecting material and separating material components**
Vorrichtung zum Einspritzen eines Stoffs und zur Scheidung der Bestandteile eines Gemisches
Dispositif pour l'injection d'un matériau et pour séparer les composants d'un mélange

(43) Date of publication of application: 01.08.2007
(73) Proprietor: Stryker Trauma GmbH, 24232 Schönkirchen/Kiel (DE)
(72) Inventor: Speitling, Andreas, 24149 Kiel (DE)
(74) Representative: Dilg, Andreas

(56) References cited:
- WO-A-2004/039439
- CH-A- 663 834
- DE-A- 3 425 566
- DE-A- 3 609 672
- DE-A- 10 353 919
- DE-U- 20 216 631
- US-A1- 2003 024 891

## Description

The invention relates to a device particularly for injecting bone cement into an object.
The invention may be used in a method of injecting bone cement into an object.
Moreover, the invention relates to a medical arrangement.

Also disclosed but not forming part of the invention are a device for separating at least one of a plurality of components of a multi-component sample and

a method of using a device for separating at least one of a plurality of components of a biological multi-component sample.

Bone cement syringes may be used for injecting bone cement in a fracture in order to promote convalescence of a patient suffering from a bone fracture.

DE 19848479 A1 discloses a method of and a device for inserting fluidic bone cement in an intermediate space between a prothesis inserted in a hollow space of a bone and the wall of the hollow space. During such a method, a homogeneous bone cement coat is generated and simultaneously the danger of injuries is reduced by inserting the bone cement via a conduit system with a constant volume stream and to break the supply when the pressure in the supplied bone cement exceeds a particular maxiumum value.

DE 3609672 A1 discloses an evacuatable bone cement syringe comprising a container for receiving the bone cement prior to its application, and comprising a pressure generating apparatus for precompressing the bone cement in the container. A bell comprising a vacuum tube is placed over the container and held in place by a flange so that the gases which escape during the process of precompression can be sucked off. In this way it is possible to reduce the porosity of the bone cement which is being applied.

DE 4243877 A1 discloses, for air-free mixing of bone cement and subsequent application thereof with accurate pressure, that the bone cement be mixed in a closed vacuum which is generated by changing the volume either of the mixing cup or of a vessel surrounding the latter. A pressure applicator with an air reservoir is used to apply the cement with a controllable pressure, so that volume-controlled expulsion of the cement at predeterminable pressures is possible.

DE 3425566 A1 discloses a device and a process for mixing and applying bone cement. The device comprises a sealable container wherein the bone cement, prior to its application, is prepressurized at an adjustable pressure and from which it is subsequently applied at a controllable pressure. The device and the process of the invention allow bubble formation in the bone cement to be suppressed and to achieve the desired stratification of the bone cement around the prosthesis during application. The device of DE 3425566 A1, on which the preamble of claim 1 is based, includes vent holes and a vent closure element.

It is an object of the invention to allow for an efficient handling of a viscous or multi-component substance.

In order to achieve the object defined above, a device for and a method of injecting bone cement into an object, as well as a medical arrangement according to the independent claims are provided.

According to the invention, a device for injecting viscous material (particularly bone cement) into an object is provided, the device comprising a container adapted for receiving viscous material (particularly bone cement), an inlet adapted for inserting viscous material (particularly bone cement) in the container, an outlet adapted for injecting viscous material (particularly bone cement) from the container into the object, and at least one vent hole formed in the container and being adapted for removing gas from an interior of the container.

According to exemplary embodiment of the invention, a medical arrangement is provided, the medical arrangement comprising a device having the above-mentioned features for injecting viscous material (particularly bone cement) into an object, and comprising a fixture adapted for accommodating the device.

According to a use of the invention, a method of injecting viscous material (particularly bone cement) into an object is provided, the method comprising inserting viscous material (particularly bone cement) in an inlet of a container, injecting viscous material (particularly bone cement) from the container via an outlet into the object, and removing gas from an interior of the container via at least one vent hole formed in the container.

A device for separating at least one of a plurality of components of a multi-component sample is also provided, the device comprising a container adapted for receiving the multi-component sample, an inlet adapted for inserting the multi-component sample in the container, a plunger adapted to act on the multi-component sample in the container upon actuation of the plunger, and at least one vent hole formed in the container and being adapted for separating the at least one of the plurality of components of the multi-component sample based on a varying permeability of the at least one vent hole for the different components of the multi-component sample.

A method of using a device having the above mentioned features for separating at least one of a plurality of components of a biological multi-component sample is also provided.

The term "viscous material" may particularly cover any liquid containing material, like pure liquid, a mixture of a liquid and a solid component, a mixture of a liquid and a gaseous component, a mixture of a liquid and a solid and a gaseous component, etc. Particularly, this term may cover any form of bone cement.

Thus, according to the invention, one or more ventilation holes are formed in the container and may serve to remove gaseous components, for instance embedded in bone cement, from the container before or during injecting the bone cement from the container into the object. By taking this measure, the quality of the treatment and the homogeneity of the inserted bone cement may be significantly improved and the convalescence of a patient being subject of such a treatment may be promoted. By forming such a vent hole in the container, it may be securely ensured that the bone cement does essentially only consist of solid and/or liquid components with reduced or minimal disturbing inclusions of gas, like air.

In accordance with the foregoing, according to the invention, an application body having a ventilation feature is be provided. Such an application body may be used for injecting bone cement (for instance bioresorbable bone cement comprising calcium phosphate ceramics or the like) or other materials into a region of a bone fraction of a patient.

Bone cement may be a composition of two components, namely a powder component (for instance made of a monomer or a polymer or a ceramics) and a liquid component. After having prepared the two-component bone cement, the bone cement may be filled in a container (for instance a syringe) which may be located - for instance with the help of a fixture (for example a plate having a beveled bore adapted for receiving the syringe, or a correspondingly designed support frame) - tilted with respect to a horizontal and with respect to a vertical axis. Such a positioning may reduce undesired gaseous bubbles inside the bone cement. However, solely by a beveled fill-in geometry, it may not entirely be preventable that bubbles remain within the bone cement and/or between a bottom of the container or syringe and the lower end of a bone cement cluster. Such bubbles or gaseous portions may have the effect that liquid (e.g. water) is decomposed or unmixed from the bone cement. This may dehydrate the bone cement and may modify the viscosity of the bone cement in an undesired way. Thus, deaerating the bone cement and/or a portion between a bottom of the syringe and the lower end of a bone cement cluster using the ventilation structures formed in the syringe may significantly improve the quality of the prepared cement.

It may be desirable to insert such a bone cement material in a minimum invasive manner for augmenting dislocated fractures. In such a context, it is desirable that the position of the fracture itself is not opened. A surgeon may therefore carry out the application in a remote position with regard to the fraction using a syringe with an appropriate designed cannula.

It may happen that gaseous embeddings or inclusions like air bubbles or blebs are present in addition to the cement inside of the syringe. This may have an influence on the homogeneity and the solidity of the applied cement body. According to a method making use of the invention, such gaseous embeddings may be avoided or may be removed from the cement when the latter is to be transported from the syringe into a body. The gas may be eliminated from the interior of the cement by, for instance, a mechanical pressure acting on the cement infill and may escape from the container through the vent hole(s) formed therein.

For refilling prepared cement from a crucible or pot into a syringe, it may happen that air blebs are maintained within the cement. According to an exemplary embodiment of the invention, such blebs may be removed from the cement through the vent hole(s) which may be formed in a wall of the syringe. For instance, when a plunger is inserted in a container of the syringe so as to compress the cement and to press the cement through a tip at an end portion of the syringe to a destination, air emitted from the compressed cement (particularly from a section between a surface of the cement and the plunger) may escape through the at least one vent hole.

This may have a consequence that a proper stability or solidness of the cement after application into the fracture may be obtained, whereas pores or blebs in the cement may reduce such a solidity.

Furthermore, embodiments of the invention may overcome also the following problem. A gas cushion within bone cement can have the effect that, when injecting the bone cement (particularly when injecting a bone cement made of calcium phosphate ceramics), a decomposition or a demixing of solid and fluidic components of the cement may occur. This may have the consequence that the viscosity of the cement is increased in the syringe directly, and that the injection of the bone cement is no more possible, even with a very large pressure.

By removing gas from the cement through the vent holes in the syringe such problems may be overcome. For this purpose, one or more ventilation bores may be provided at a circumferential portion of the syringe. The position (for instance height) of the bore or bores may essentially correspond to a respective desired filling amount or level of the syringe. If necessary, the position (for instance height) of the bore(s) can also be below a desired default filling amount or level.

The bone cement, particularly a calcium phosphate ceramic bone cement, can be inserted into an inclinedly oriented syringe (for instance inclined with an angle of essentially 15° with respect to a horizontal plane). This may be performed in order to maintain the volume of the gas insertions within the cement as small as possible. The ventilation bore(s) may therefore be provided on an upper side of the container of the syringe with respect to a horizontal plane. By taking this measure, a gas cushion inside the container may disappear when a plunger of the syringe is pressed down, for instance by manpower or powered and controlled by a machine.

Optionally, the vent bore can be closed after the gas has disappeared from the interior of the syringe. To close the vent hole or holes, a turnable ring, slider or the like may be turned in order to stop an undesired discharge of the cement through the bore(s).

Next, further exemplary embodiments of the invention will be described.

In the following, further exemplary embodiments of the device for injecting bone cement into an object will be described. However, these embodiments also apply for the medical arrangement.

The device may be adapted for injecting bone cement into a bone or into a fracture of a bone. Thus, the device, particularly a tip of the device, may be made of a material and may be dimensioned in such a manner that it is made possible to convey bone cement material through such a tip. Particularly, bioresorbable bone cement materials like bone cement made of calcium phosphate ceramics (but not limited to such materials) should be treatable by means of the device according to exemplary embodiments of the invention. Bone cement composites may be prepared, like acrylic cement, and may contain a range of powders such as monocalcium phosphate, tricalcium phosphate and calcium carbonate, which may be mixed in a solution of sodium phosphate. Such cement materials may be produced without polymerization. Also injection of calcium phosphate cement is feasible and may provide for a high compressive strength.

The container of the device may be a hollow tube. In other words, the circumference of the container may be circular, and a circular hole may be provided in the interior of the container. A plunger can be guided through the circular hole. A pressure acting on the plunger may release material from an inside of the container through the outlet under the influence of the pressure generated by the compressed plunger.

The inlet may be formed at a first end portion of the hollow tube, and the outlet may be formed at a second (opposing) end portion of the hollow tube. A sectional area of the opening of the outlet may be significantly smaller than a sectional area of the opening of the inlet so that an easy insertion of the cement inside the container and a precise definition of the destination location of cement emitted via the outlet may be ensured.

The outlet may be formed as a hollow tip connected to the container. The container may be manufactured from a plastics or a glass material, and the hollow tip may be made of a metallic material, for instance as some kind of needle with an internal bore.

Furthermore, the at least one vent hole of the device may be formed in the container at a position between the inlet and the outlet. Therefore, when the cement is compressed by the plunger, gas emitted from the compressed viscous cement may be dissipated through the at least one vent hole.

Particularly, the at least one vent hole may be formed at a lateral position of the container. It is also possible that a plurality of vent holes are formed along a longitudinal and/or along a circumferential extension of the container at appropriate positions so that the path along which the gas flows between the cement and the corresponding ventilation hole is kept short.

The at least one vent hole may be formed at a position of the container which is below of or which essentially corresponds to a bone cement filling level of the container. Taking this measure may promote the functionality of the system, since this may ensure rapid removal of the gas from the interior of the container. The position at which the vent hole(s) may be provided, calculated from the outlet end portion of the container, may be such that the filling volume below this vent hole is in the dimension of essentially 2.5 cm³ to 20 cm³.

The device may further comprise a plunger adapted to be insertable in the container so as to inject bone cement in the object (for instance a position close to a bone fracture) in an operation state in which the plunger is pushed inside the container. Such a plunger may have an end portion which has essentially the same or a similar internal shape as the container and which acts on/in the direction of the cement when a human user generates a force acting on an end portion of a connection rod between the end portion of the plunger and a handle of the plunger. In other words, the cement within the container may be compressed in a manner as in a medical syringe, for instance for extracting blood from a human body or for inserting a medication inside a human body.

Beyond this, the device comprises a vent hole closure element adapted to be operated between a first operation state in which the vent hole closure element covers the at least one vent hole and a second operation state in which the vent hole closure element is free from covering the at least one vent hole. Such a vent hole closure element is a piece of material which is selectively shifted in front of the vent hole or in front of at least a part of the vent holes so as to avoid that cement or individual components thereof is/are undesirably emitted through the vent holes, for instance after the gas has been removed from the inside of the container. Such a vent hole closure element may be located outside or inside of the container, and may be, for instance, be operated in accordance with an operation of a plunger. In other words, when a plunger is operated in a special manner, for instance is rotated, the vent holes may be opened or closed by being selectively covered by the vent hole closure element.

It is also possible to operate the vent hole closure element between a closed and an open position by pressing a button or by actuating a lever mechanism.

The vent hole closure element comprises a tube having a longitudinal slit. When the slit is brought in alignment with the vent holes, the vent holes are open and have the function to remove gas from inside of the container to the outside. When the tube having the longitudinal slit is turned in such a manner that the slit is brought out of alignment with the vent holes and the tube covers the vent holes, any material inside of the container, also cement, is prevented from being removed from the container via the vent holes.

Therefore, the vent hole closure element may be adapted to be turnable outside or inside the container, for instance to selectively open or close at least a part of the vent hole(s) under the control of a user or under the control of a machine. The vent hole closure element may be adapted as a sleeve with a slit which sleeve may be shifted about an outer surface of the tube of the syringe and may be adjusted manually by a human user so as to selectively cover or uncover the vent hole(s).

In the case of more than one (for instance ten) vent holes being provided along the tube, the vent holes may be located along a curved line. This may ensure that individual ones of the vent holes may be closed or opened independently from one another, for instance using a slitted or perforated vent hole closure sleeve or tube. By successively turning such a sleeve, one vent hole may be closed after the other.

The device may comprise a bone cement drain member adapted for draining off bone cement passed through the at least one vent hole. In a scenario in which gas is removed from the container or from the cement, it may happen that (undesirably) some bone cement material is pressed through the vent hole(s) out of the container. To avoid any contamination of the environment with such bone cement material, the vent hole(s) may be coupled with some kind of conduit or waste container, in which the removed bone cement may be collected or accumulated.

Particularly, the device may be adapted as a syringe of a type similar as used for conventional medical treatment. A syringe may be a small hollow tube used for injecting or withdrawing cement material. It may be attached to a needle in order to inject material into a destination. Thus, a syringe may be an instrument for introducing fluids or solids or mixtures thereof into or withdrawing them. Syringes may comprise a needle attached to a hollow cylinder that is fitted with a sliding plunger. The downward movement of the plunger injects material located inside of the container into a destination object. A bone cement container of the syringe may be manufactured from a plastics material like nylon.

The vent hole or the vent holes may have a dimension which is large enough for enabling an efficient gas flow through the holes and which is small enough for essentially preventing a bone cement flow through the holes. The different values of viscosity of air and of bone cement may be a cause for the differing flow characteristics. A possible linear dimension of a vent hole is in the range between 0.01 mm and 2 mm, particularly 0.1 mm and 2 mm, more particularly in the range between 0.2 mm and 1 mm.

In the following, further exemplary embodiments of the medical arrangement will be described. However, these embodiments also apply for the device for and for the method of injecting bone cement into an object.

The fixture of the medical arrangement may be adapted for accommodating the device in such a manner that the container of the device is accommodated in an angled manner with respect to a horizontal plane. When the device is received in a suitably shaped hole or recess provided in the fixture, the syringe may be oriented bevelled or inclined with respect to a horizontal plane and/or with respect to a vertical vector which is oriented perpendicular to the horizontal plane. When the device is accommodated in the fixture, an injection of cement into the device may be simplified so that the system may be operated in a user-friendly manner. This particularly holds when the angle between the container in the horizontal plane is in the range between essentially 15° and essentially 30°. With such an injection configuration, it may be possible to reduce the amount of gas included in the cement.

Particularly, the fixture may be adapted to detachably accommodate the device. In other words, after having filled in cement into the container of the device, the device may be removed from the fixture and may be brought to a position in which cement inside the device may be injected into a bone fracture.

In the following, a further exemplary method of injecting bone cement into an object will be described.

The bone cement may be injected into the inlet of the container in an operation state in which the device is arranged angled with respect to a horizontal plane. This may have the effect that during the insertion of cement into the container, the amount of gas which is undesirably included in the cement may be kept small.

In the following, further exemplary embodiments of the device for separating at least one of a plurality of components of a multi-component sample will be described. However, these embodiments also apply for the corresponding method of use.

Such a device may be capable to separate different fractions of a mixture of these fractions (for instance of different aggregation states like liquid and solid), particularly due to different viscosity values of these fractions. For example, it may happen that a mixture of blood, bone chippings and bone cement particles is present. Compressing such a mixture in a container having holes by a plunger or the like may press particles of a small dimension (below a threshold value defined by the bore size) through the bore(s), whereas larger particles remain in the container. Thus, the different components may be separated based on different sizes and/or viscosity with low effort and high efficiency. This may allow, for example, to separate lipid components and/or protein components of a blood sample from other components.

The device may comprise a plurality of vent holes formed in the container. These may be designed in a manner (alignment, dimension, position, etc.) to promote the separation functionality.

For instance, the plurality of vent holes may be provided in different sizes, in accordance with different components in the sample to be analyzed.

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.

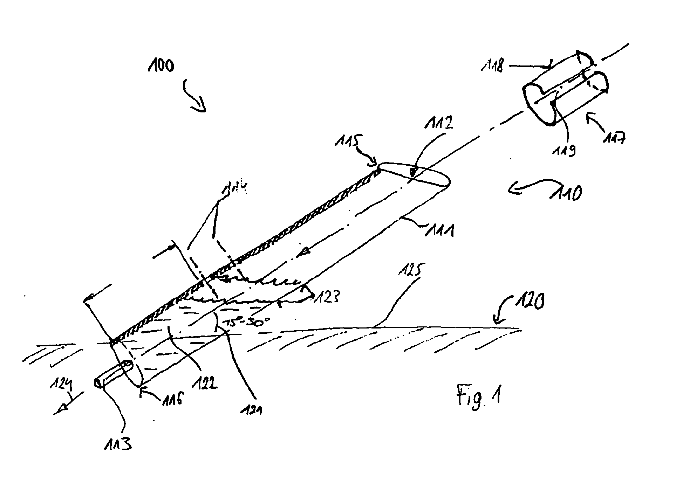

Fig. 1 shows a medical arrangement according to an exemplary embodiment of the invention.

The illustration in the drawing is schematically.

In the following, referring to **Fig. 1**, a medical arrangement 100 according to an exemplary embodiment of the invention will be described.

The medical arrangement 100 comprises a device for injecting bone cement 122 into a bone fracture (not shown). Furthermore, the medical arrangement 100 comprises a fixture 120 having a bore provided therein for detachably receiving the device 110.

The device 110 is adapted for injecting bone cement 122 into a bone fracture and comprises a tubular container 111 adapted for receiving the bone cement 122, comprises an inlet 112 adapted for inserting the bone cement 122 in the container 111, comprises a tip-like outlet 113 for injecting bone cement 122 from the container 111 into the bone fracture, and comprises two vent holes 114 formed in a wall of the tubular container 111 and being adapted for removing gas from an interior of the container 111, particularly for removing gas which is located inside of the cement 122.

As can be taken from Fig. 1, the inlet 112 is formed at a first end portion 115 of the hollow tube 111, and the outlet 112 is formed at a second end portion 116 of the hollow tube 111. The outlet 112 is provided as a hollow tip tightly connected to the container 111.

The vent holes 114 are formed in a wall of the container 111 at a position between the inlet 112 and the outlet 113. Particularly, the vent holes 114 are formed at a lateral position of the container 111, that is to say at a circumferential wall of the container 111. The two vent holes 114 are spaced apart from one another along a longitudinal extension of the container 111.

As can be taken from Fig. 1, the vent holes 114 are formed at positions of the container 111 which is below of or essentially corresponds to a bone cement filling level 123 of the container 111. Two cement filling levels 123 are indicated in Fig. 1, which are both possible and which may both enable to remove gas inside of the cement 122 through at least one of the vent holes 114. The default cement filling level 123 may be in the order of magnitude of 9 cm³ to 10 cm³.

The device 110 may further comprise a plunger (not shown in Fig. 1) adapted to be insertable in the hollow cylinder 111 so as to inject bone cement 122 in a bone fracture in an operation state of the device 110 in which a plunger (not shown) is pushed inside the container 111.

Furthermore, a vent hole closure element 117 is provided as a tubular ring 118 having a longitudinal slit 119. The vent hole closure element 117 is shaped so as to be insertable in the container 111. The vent hole closure element 117, when located inside of the container 111, can be operated between a first operation state in which the vent hole closure element 117 covers the vent holes 114, and a second operation state in which the vent hole closure element 117 does not cover the vent holes 114. In the first operation state, gas and small amounts of cement 122 may escape through the holes 114, and in the second operation state cement 122 may be securely prevented from being undesirably pressed outside of the container 111 through the holes 114.

The vent hole closure element 117 is turnable inside the container 111 so that in one operation state the longitudinal slit 119 may be in alignment with the holes 114 so as to allow gas to be transported from the inside of the container 111 to the outside of the container 111. When the vent hole closure element 117 is turned around the symmetry axis of the tubular element 111 and of the ring 118, the slit 119 may be brought out of alignment with the holes 114 so as to prevent cement 121 from being pressed through the holes 114 by the wall of the ring 118. The vent hole closure element 117 may also be denoted as a locking ring.

An arrow 124 indicates the direction in which the cement is released out of the tip 113.

As can further be taken from Fig. 1, a horizontal plane 125, that is a surface of the planar fixture 120 and the symmetry axis of the tube 111 are inclined with respect to one another by an angle 121 of 15° to 30°, which may be defined by a hole in the fixture 120 defining the inclination of the syringe 110 with respect to the fixture 120. This inclination angle is a proper angle for filling in cement via the inlet 112 in such a manner that the amount of gas inserted in the cement 122 is kept small.

It should be noted that the term "comprising" does not exclude other elements or steps and the "a" or "an" does not exclude a plurality. Also elements described in association with different embodiments may be combined in so far as the resulting combinations fall within the scope of the claims.

It should also be noted that reference signs in the claims shall not be construed as limiting the scope of the claims.

## Claims

1. A device (110) for injecting viscous material into an object, the device (110) comprising
a container (111) adapted for receiving viscous material;
an inlet (112) adapted for inserting viscous material in the container (111);
an outlet (113) adapted for injecting viscous material from the container (111) into the object;
at least one vent hole (114) formed in the container (111) and being adapted for removing gas from an interior of the container (111),
a vent hole closure element (117) adapted to be operated between a first operation state in which the vent hole closure element (117) covers at least one of the at least one vent hole (114) and a second operation state in which the vent hole closure element (117) is free from covering the at least one of the at least one vent hole (114), **characterised in that**
the vent hole closure element (117) comprises a tube (118) having a longitudinal slit (119).

2. The device (110) according to claim 1,
wherein the device (110) is adapted for injecting bone cement as the viscous material into a bone or into a fracture of a bone.

3. The device (110) according to claim 1 or any one of the above claims,
wherein the container (111) is a hollow tube.

4. The device (110) according to claim 3,
wherein the inlet (112) is formed at a first end portion (115) of the hollow tube (111).

5. The device (110) according to claim 3 or any one of the above claims,
wherein the outlet (112) is formed at a second end portion (116) of the hollow tube (111).

6. The device (110) according to claim 1 or any one of the above claims,
wherein the outlet (112) is formed as a hollow tip connected to the container (111).

7. The device (110) according to claim 1 or any one of the above claims,
wherein the at least one vent hole (114) is formed in the container (111) at a position between the inlet (112) and the outlet (113).

8. The device (110) according to claim 1 or any one of the above claims,
wherein the at least one vent hole (114) is formed at a lateral position of the container (111).

9. The device (110) according to claim 1 or any one of the above claims,
wherein a plurality of vent holes (114) are formed along a longitudinal extension of the container (111).

10. The device (110) according to claim 1 or any one of the above claims,
wherein the at least one vent hole (114) is formed at a position of the container (111) which is below of or essentially corresponds to a viscous material filling level of the container (111).

11. The device (110) according to claim 1 or any one of the above claims,
comprising a plunger adapted to be insertable in the container (111) so as to inject viscous material in the object in an operation state in which the plunger is pushed inside the container (111).

12. The device (110) according to claim 1 or any one of the above claims,
wherein the vent hole closure element (117) is adapted to be turnable outside of the container (111) or inside the container (111).

13. The device (110) according to claim 1 or any one of the above claims,
comprising a viscous material drain member adapted for draining off viscous material passed through the at least one vent hole (114).

14. The device (110) according to claim 1 or any one of the above claims,
adapted as a syringe.

15. The device (110) according to claim 1 or any one of the above claims,
wherein a plurality of vent holes (114) are formed along one of the group consisting of a linear, a helical and a spiral trajectory along the container (111).

16. A medical arrangement (100), the medical arrangement (100) comprising
a device (110) according to claim 1 or any one of the above claims for injecting viscous material into an object;
a fixture (120) adapted for accommodating the device (110).

17. The medical arrangement (100) according to claim 16,
wherein the fixture (120) is adapted for accommodating the device (110) in such a manner that the container (111) of the device (110) is accommodated in an angled orientation with respect to a horizontal plane.

18. The medical arrangement (110) according to claim 17,
wherein an angle (121) between the container (111) and the horizontal plane is in the range between essentially 15° and essentially 30°.

19. The medical arrangement (100) according to claim 16 or any one of the above claims,
wherein the fixture (120) is adapted for detachably accommodating the device (110).

## Patentansprüche

1. Vorrichtung (110) zum Injizieren von viskosem Material in einen Gegenstand, wobei die Vorrichtung (110) Folgendes umfasst:
einen Behälter (111), der zum Aufnehmen von viskosem Material angepasst ist;
einen Einlass (112), der zum Einbringen von viskosem Material in den Behälter (111) angepasst ist;
einen Auslass (113), der zum Injizieren von viskosem Material vom Behälter (111) in den Gegenstand angepasst ist;
mindestens ein Entlüftungsloch (114), das im Behälter (111) gebildet ist und zum Abführen von Gas aus einem Innenraum des Behälters (111) angepasst ist;
ein Entlüftungsloch-Schließelement (117), das angepasst ist, um zwischen einem ersten Betriebszustand, in dem das Entlüftungsloch-Schließelement (117) mindestens eines des mindestens einen Entlüftungslochs (114) bedeckt, und einem zweiten Betriebszustand zu arbeiten, in dem das Entlüftungsloch-Schließelement (117) das mindestens eine des mindestens einen Entlüftungslochs (114) nicht bedeckt, **dadurch gekennzeichnet, dass:**
das Entlüftungsloch-Schließelement (117) ein Rohr (118) umfasst, das einen Längsschlitz (119) aufweist.

2. Vorrichtung (110) nach Anspruch 1, wobei die Vorrichtung (110) zum Injizieren von Knochenzement als das viskose Material in einen Knochen oder in eine Knochenfraktur angepasst ist.

3. Vorrichtung (110) nach Anspruch 1 oder einem der vorhergehenden Ansprüche, wobei der Behälter (111) ein hohles Rohr ist.

4. Vorrichtung (110) nach Anspruch 3, wobei der Einlass (112) aus einem ersten Endabschnitt (115) des hohlen Rohrs (111) gebildet ist.

5. Vorrichtung (110) nach Anspruch 3 oder einem der vorhergehenden Ansprüche, wobei der Auslass (112) aus einem zweiten Endabschnitt (116) des hohlen Rohrs (111) gebildet ist.

6. Vorrichtung (110) nach Anspruch 1 oder einem der vorhergehenden Ansprüche, wobei der Auslass (112) als eine hohle Spitze gebildet ist, die mit dem Behälter (111) verbunden ist.

7. Vorrichtung (110) nach Anspruch 1 oder einem der vorhergehenden Ansprüche, wobei das mindestens eine Entlüftungsloch (114) im Behälter (111) an einer Stelle zwischen dem Einlass (112) und dem Auslass (113) gebildet ist.

8. Vorrichtung (110) nach Anspruch 1 oder einem der vorhergehenden Ansprüche, wobei das mindestens eine Entlüftungsloch (114) an einer seitlichen Stelle des Behälters (111) gebildet ist.

9. Vorrichtung (110) nach Anspruch 1 oder einem der vorhergehenden Ansprüche, wobei mehrere Entlüftungslöcher (114) entlang einer Längsausdehnung des Behälters (111) gebildet sind.

10. Vorrichtung (110) nach Anspruch 1 oder einem der vorhergehenden Ansprüche, wobei das mindestens eine Entlüftungsloch (114) an einer Stelle des Behälters (111) gebildet ist, die unter einem Füllstand des viskosen Materials des Behälters (111) liegt oder ihm im Wesentlichen entspricht.

11. Vorrichtung (110) nach Anspruch 1 oder einem der vorhergehenden Ansprüche, die einen Kolben umfasst, der angepasst ist, in den Behälter (111) eingeführt werden zu können, um in einem Betriebszustand, in dem der Kolben in den Behälter (111) geschoben wird, viskoses Material in den Gegenstand zu injizieren.

12. Vorrichtung (110) nach Anspruch 1 oder einem der vorhergehenden Ansprüche, wobei das Entlüftungsloch-Schließelement (117) angepasst ist, um außerhalb des Behälters (111) oder innerhalb des Behälters (111) drehbar zu sein.

13. Vorrichtung (110) nach Anspruch 1 oder einem der vorhergehenden Ansprüche, die ein Element zum Ablassen von viskosem Material umfasst, das zum Ablassen des durch das mindestens eine Entlüftungsloch (114) durchgeleiteten viskosen Materials angepasst ist.

14. Vorrichtung (110) nach Anspruch 1 oder einem der vorhergehenden Ansprüche, die als eine Spritze angepasst ist.

15. Vorrichtung (110) nach Anspruch 1 oder einem der vorhergehenden Ansprüche, wobei mehrere Entlüftungslöcher (114) entlang einer linearen, einer schraubenförmigen oder einer spiralförmigen Trajektorie entlang dem Behälter (111) ausgebildet sind.

16. Medizinische Einrichtung (100), wobei die medizinische Einrichtung (100) Folgendes umfasst:
eine Vorrichtung (110) nach Anspruch 1 oder einem der vorhergehenden Ansprüche zum Injizieren von viskosem Material in einen Gegenstand;
eine Halterung (120), die zum Aufnehmen der Vorrichtung (110) angepasst ist.

17. Medizinische Einrichtung (100) nach Anspruch 16, wobei die Halterung (120) derart zum Aufnehmen der Vorrichtung (110) angepasst ist, dass der Behälter (111) der Vorrichtung (110) in einer abgewinkelten Ausrichtung in Bezug auf eine horizontale Ebene aufgenommen ist.

18. Medizinische Einrichtung (100) nach Anspruch 17, wobei der Winkel (121) zwischen dem Behälter (111) und der horizontalen Ebene in einem Bereich zwischen im Wesentlichen 15° und im Wesentlichen 30° liegt.

19. Medizinische Einrichtung (100) nach Anspruch 16 oder einem der vorhergehenden Ansprüche, wobei die Halterung (120) zur abnehmbaren Aufnahme der Vorrichtung (110) angepasst ist.

## Revendications

1. Dispositif (110) destiné à injecter une matière visqueuse dans un objet, le dispositif (110) comportant
un conteneur (111) adapté pour recevoir la matière visqueuse,
une entrée (112) adaptée pour introduire la matière visqueuse dans le conteneur (111),
une sortie (113) adaptée pour injecter la matière visqueuse à partir du conteneur (111) dans l'objet,
au moins un trou d'évent (114) formé dans le conteneur (111) et adapté pour retirer du gaz d'un espace intérieur du conteneur (111),
un élément d'obturation de trou d'évent (117) adapté pour être actionné entre un premier état de fonctionnement dans lequel l'élément d'obturation de trou d'évent (117) recouvre au moins un parmi les au moins un trou d'évent (114), et un second état de fonctionnement dans lequel l'élément d'obturation de trou d'évent (117) est libre de recouvrir le au moins un parmi les au moins un trou d'évent (114),
**caractérisé en ce que** l'élément d'obturation de trou d'évent (117) comporte un tube (118) ayant une fente longitudinale (119).

2. Dispositif (110) selon la revendication 1,
dans lequel le dispositif (110) est adapté pour injecter un ciment osseux en tant que matière visqueuse dans un os ou dans une fracture d'os.

3. Dispositif (110) selon la revendication 1 ou l'une quelconque des revendications ci-dessus,
dans lequel le conteneur (111) est un tube creux.

4. Dispositif (110) selon la revendication 3,
dans lequel l'entrée (112) est formée sur une première partie d'extrémité (115) du tube creux (111).

5. Dispositif (110) selon la revendication 3 ou l'une quelconques des revendications ci-dessus,
dans lequel la sortie (112) est formée sur une seconde partie d'extrémité (116) du tube creux (111).

6. Dispositif (110) selon revendication 1 ou l'une quelconque des revendications ci-dessus,
dans lequel la sortie (112) a la forme d'un embout creux relié au conteneur (111).

7. Dispositif (110) selon la revendication 1 ou l'une quelconque des revendications ci-dessus,
dans lequel le au moins un trou d'évent (114) est formé dans le conteneur (111) sur une position située entre l'entrée (112) et la sortie (113).

8. Dispositif (110) selon la revendication 1 ou l'une quelconque des revendications ci-dessus,
dans lequel le au moins un trou d'évent (114) est formé sur une position latérale du conteneur (111).

9. Dispositif (110) selon la revendication 1 ou l'une quelconque des revendications ci-dessus,
dans lequel une pluralité de trous d'évent (114) est formée le long d'une extension longitudinale du conteneur (111).

10. Dispositif (110) selon la revendication 1 ou l'une quelconque des revendications ci-dessus,
dans lequel le au moins un trou d'évent (114) est formé sur une position du conteneur (111) qui est sous un niveau de remplissage de matière visqueuse du conteneur (111), ou qui correspond sensiblement à celui-ci.

11. Dispositif (110) selon la revendication 1 ou l'une quelconque des revendications ci-dessus,
comportant un poussoir adapté pour pouvoir être introduit dans le conteneur (111) de manière à injecter de la matière visqueuse dans l'objet dans un état de fonctionnement dans lequel le poussoir est poussé à l'intérieur du conteneur (111).

12. Dispositif (110) selon la revendication 1 ou l'une quelconque des revendications ci-dessus,
dans lequel l'élément d'obturation de trou d'évent (117) est adapté pour pouvoir être tourné à l'extérieur du conteneur (111) ou à l'intérieur du conteneur (111).

13. Dispositif (110) selon la revendication 1 ou l'une quelconque des revendications ci-dessus,
comportant un élément d'évacuation de matière visqueuse adapté pour évacuer la matière visqueuse qui est passée à travers le au moins un trou d'évent (114).

14. Dispositif (110) selon la revendication 1 ou l'une quelconque des revendications ci-dessus,
adapté sous la forme d'une seringue.

15. Dispositif (110) selon la revendication 1 ou l'une quelconque des revendications ci-dessus,
dans lequel une pluralité de trous d'évent (114) est formée le long du groupe constitué d'une trajectoire linéaire, hélicoïdal et en spirale le long du conteneur (111).

16. Agencement médical (100), l'agencement médical (100) comportant
un dispositif (110) selon la revendication 1 ou l'une quelconque des revendications ci-dessus pour injecter de la matière visqueuse dans un objet,
un dispositif de fixation (120) adapté pour recevoir le dispositif (110).

17. Agencement médical (100) selon la revendication 16,
dans lequel le dispositif de fixation (120) est adapté pour recevoir le dispositif (110) de manière à ce que le conteneur (111) du dispositif (110) soit reçu dans une orientation oblique par rapport à un plan horizontal.

18. Agencement médical (110) selon la revendication 17,
dans lequel un angle (121) entre le conteneur (111) et le plan horizontal est dans la plage comprise entre sensiblement 15 ° et sensiblement 30 °.

19. Agencement médical (100) selon la revendication 16 ou l'une quelconque des revendications ci-dessus,
dans lequel le dispositif de fixation (120) est adapté pour recevoir le dispositif (110) de manière amovible.
